(19) Europäisches Patentamt
European Patent Office
Office européen des brevets

(11) EP 4 674 285 A1

(12) # EUROPEAN PATENT APPLICATION
published in accordance with Art. 153(4) EPC

(43) Date of publication:
**07.01.2026 Bulletin 2026/02**

(21) Application number: **24763892.7**

(22) Date of filing: **27.02.2024**

(51) International Patent Classification (IPC):
***A23L 33/10*** *(2016.01)* ***A61K 31/7048*** *(2006.01)*
***A61P 9/00*** *(2006.01)*

(52) Cooperative Patent Classification (CPC):
**A23L 33/10; A61K 31/7048; A61P 9/00**

(86) International application number:
**PCT/JP2024/006981**

(87) International publication number:
**WO 2024/181413 (06.09.2024 Gazette 2024/36)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **02.03.2023 JP 2023032193**

(71) Applicant: **Toyo Sugar Refining Co., Ltd.**
**Tokyo 1030006 (JP)**

(72) Inventors:
- **HASHIZUME, Yushi**
  **Ichihara-shi, Chiba 290-0046 (JP)**
- **TANDIA, Mahamadou**
  **Noda-shi, Chiba 278-0027 (JP)**

(74) Representative: **Dehns**
**10 Old Bailey**
**London EC4M 7NG (GB)**

(54) **VASCULAR ENDOTHELIAL FUNCTION IMPROVING AGENT**

(57) An object of the present invention is to provide an excellent vascular endothelial function improving agent containing hesperidin and rutin. The present invention includes a vascular endothelial function improving agent containing alpha-glucosyl hesperidin and alpha-glucosyl rutin.

EP 4 674 285 A1

## Description

### Technical Field

The present invention relates to a vascular endothelial function improving agent.

### Background Art

Vascular endothelial cells are cells in the innermost layer of blood vessels surrounding the whole body, and play an important role in maintaining the health of blood vessels. It is known that the function of vascular endothelial cells, that is, the vascular endothelial function deteriorates with, for example, disturbance of lifestyle, such as smoking, drinking, dietary habit, and exercise, as well as overwork, stress, and aging. Evaluation of the vascular endothelial function can be conducted by a flow mediated dilation (FMD) test. Usually, when the FMD value is 7% or more, the vascular endothelial function is evaluated to be normal. When the FMD value is 4% or more and less than 7%, the vascular endothelial function is evaluated to be borderline. When the FMD value is less than 4%, the vascular endothelial function is suspected to be abnormal. Impairment of the vascular endothelial function may lead to thickening or hardening of the blood vessel wall.

Under such circumstances, studies for the purpose of improving blood circulation have been conducted using vitamin P. For example, Patent Literature 1 discloses a blood circulation improving agent containing predetermined amounts of transglycosylated vitamin P and trehalose. Patent Literature 2 discloses an elastase activity inhibitor containing vitamin P.

### Citation List

### Patent Literatures

Patent Literature 1: JP 4295840 B2
Patent Literature 2: WO 2009/116450 A1

### Summary of Invention

### Technical Problems

In Patent Literature 1, alleviation of muscle stiffness is evaluated using a poultice containing transglycosylated hesperidin as transglycosylated vitamin P and crystalline trehalose that is in an amount of 5 times or more the amount of the transglycosylated hesperidin. The literature suggests that a blood circulation improving action of the transglycosylated vitamin P was promoted by trehalose. In addition, in Patent Literature 2, a blood circulation promoting effect of transglycosylated naringin, transglycosylated hesperidin, and transglycosylated rutin without trehalose is studied.

However, in the above literatures, studies and effects of vitamin P compounds on the vascular endothelial function are insufficient, and there is still room for improvement.

An object of the present invention is to provide an excellent vascular endothelial function improving agent containing hesperidin and rutin.

### Solution to Problems

The present inventors have intensively studied to solve the above problems. As a result, the present inventors have found that the following configuration can solve the above problems, and have completed the present invention. Aspects of the present invention relate to, for example, the following [1] to [4].

[1] A vascular endothelial function improving agent containing:

alpha-glucosyl hesperidin; and
alpha-glucosyl rutin.

[2] The vascular endothelial function improving agent according to item [1], containing:

alpha-monoglucosyl hesperidin; and

alpha-monoglucosyl rutin.

[3] The vascular endothelial function improving agent according to item [2], wherein a content of the alpha-monoglucosyl rutin is 20 to 500 parts by mass relative to 100 parts by mass of the alpha-monoglucosyl hesperidin.
[4] A food product containing the vascular endothelial function improving agent according to any one of items [1] to [3].

Advantageous Effects of Invention

According to the present invention, it is possible to provide an excellent vascular endothelial function improving agent containing hesperidin and rutin.
In addition, according to the present invention, it is possible to maintain flexibility of blood vessels that decreases with aging.

Brief Description of Drawings

Fig. 1 shows graphs illustrating an amount of change in FMD value (%) (Fig. 1(A)) and a rate of change (%) in FMD value (%) (Fig. 1(B)) by continuous ingestion of alphaG hesperidin PA-T and alphaG rutin PS for 4 weeks.
Fig. 2 shows a graph illustrating an amount of change in FMD value (%) by continuous ingestion of alphaG hesperidin PA-T and alphaG rutin PS for 8 weeks.

Description of Embodiments

Hereinafter, a description is made of preferred embodiments for carrying out the present invention. Note that the embodiments described below represent examples of representative embodiments of the present invention, and the scope of the present invention is not narrowly interpreted by these embodiments. Note that a description of "A to B" regarding a numerical range indicates A or more and B or less unless otherwise specified. For example, a description of "1 to 5%" means 1% or more and 5% or less.
One embodiment of the present invention is a vascular endothelial function improving agent containing alpha-glucosyl hesperidin and alpha-glucosyl rutin.

<Alpha-Glucosyl hesperidin>

Alpha-Glucosyl hesperidin (also referred to as alpha glucosyl hesperidin) is a generic term for compounds in which one or more molecules of glucose are added to the hydroxyl group in the rutinose unit of hesperidin by an alpha-1,4 linkage. The alpha-glucosyl hesperidin in the present invention may be composed of only one type of the compounds having such a structure, or may be a mixture of two or more types thereof. Note that hesperidin is a compound in which beta-rutinose (6-O-alpha-L-rhamnosyl-beta-D-glucose) is bonded to the hydroxyl group at the 7 position of hesperetin, that is, a hesperetin glycoside.
Alpha-Glucosyl hesperidin can be represented by the following formula (1). In the formula (1), n is 0 or an integer of 1 or more, for example, an integer of 1 to 19.

[Chemical formula 1]

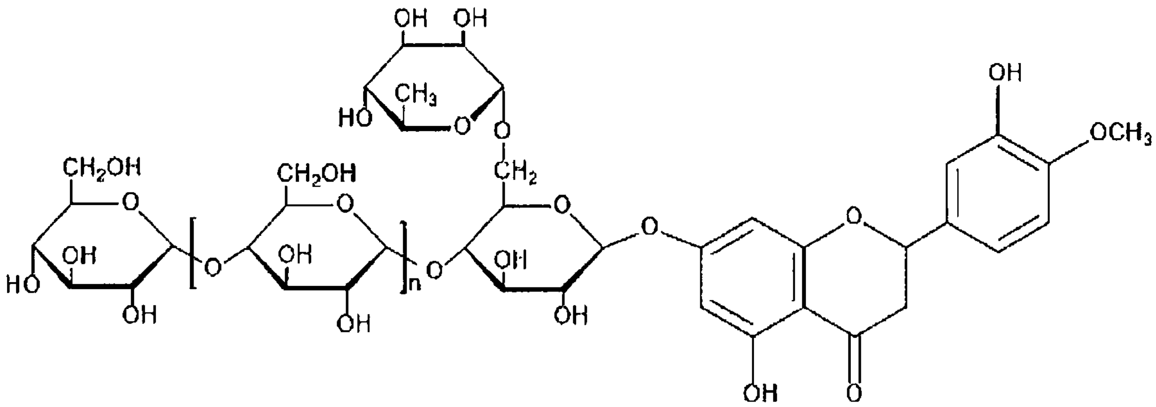

・・・(1)

Alpha-Glucosyl hesperidin is a compound contained as a main component in a material known as "enzyme-treated hesperidin" (sometimes also referred to as "transglycosylated hesperidin"). Among alpha-glucosyl hesperidin, one to which only one glucose molecule is bonded is referred to as "alpha-monoglucosyl hesperidin", and one to which two or more glucose molecules are bonded is referred to as "alpha-polyglucosyl hesperidin". That is, in the formula (1), alpha-monoglucosyl hesperidin is a compound in which n is 0, and alpha-polyglucosyl hesperidin is a compound in which n is generally 1 to 19.

Enzyme-treated hesperidin is an aggregate of compounds produced by enzyme treatment for the sugar of hesperidin, and usually contains a mixture of compounds having different numbers of molecules of glucose bonded to hesperidin, for example, a mixture composed of alpha-monoglucosyl hesperidin and alpha-polyglucosyl hesperidin. Enzyme-treated hesperidin may contain, in addition to alpha-glucosyl hesperidin, unreacted hesperidin and hesperidin derivatives other than alpha-glucosyl hesperidin, such as 7-glucosyl hesperetin (such hesperidin derivatives are also referred to as other hesperidin derivatives). However, it is preferable that the enzyme-treated hesperidin contain no hesperetin.

Examples of the enzyme treatment for the sugar of hesperidin are as follows.

[1] A glycosyltransferase is allowed to act on hesperidin in the coexistence of a sugar donor such as an alpha-glucosyl sugar compound (for example, cyclodextrin or partially decomposed starch) to add glucose to a glucose unit of hesperidin by an alpha-1,4 linkage, whereby alpha-glucosyl hesperidin is produced, and a composition containing unreacted hesperidin and alpha-glucosyl hesperidin is obtained (first enzyme-treated hesperidin).

[2] Glucoamylase or the like is allowed to act on the alpha-glucosyl hesperidin produced in the item [1] to leave only one glucose molecule while cleaving other glucose molecules from the glucose chain bonded to the glucose unit of hesperidin, whereby alpha-monoglucosyl hesperidin is produced, and a composition containing unreacted hesperidin and alpha-monoglucosyl hesperidin is obtained (second enzyme-treated hesperidin).

[3] Alpha-L-rhamnosidase is allowed to act on the unreacted hesperidin in the item [2] to cleave rhamnose contained in the rutinose unit of hesperidin, whereby 7-glucosyl hesperetin is produced, and a composition containing 7-glucosyl hesperetin and alpha-monoglucosyl hesperidin is obtained (third enzyme-treated hesperidin).

Examples of the first enzyme treatment include allowing a glycosyltransferase (for example, enzymes having the function of adding glucose to hesperidin, such as cyclodextrin glucanotransferase (CGTase, EC 2.4.1.19)) to act on hesperidin in the coexistence of an alpha-glucosyl sugar compound (for example, cyclodextrin or partially decomposed starch).

In the vascular endothelial function improving agent according to an embodiment of the present invention, in consideration of the effect or the like of the present invention, enzyme-treated hesperidin that is a composition containing alpha-glucosyl hesperidin is preferably used, or any composition of the first enzyme-treated hesperidin, the second enzyme-treated hesperidin, and the third enzyme-treated hesperidin may be used.

In consideration of the effect or the like of the present invention, the enzyme-treated hesperidin contains at least alpha-glucosyl hesperidin, and is preferably a mixture further containing one or both of hesperidin and 7-glucosyl hesperetin.

Examples of commercially available products of the enzyme-treated hesperidin include "alphaG hesperidin PS-CC" and "alphaG hesperidin PA-T" that are products of Toyo Sugar Refining Co., Ltd. "alphaG hesperidin PS-CC" contains 80 mass% or more of alpha-monoglucosyl hesperidin, and contains 7-glucosyl hesperetin. "alphaG hesperidin PA-T" contains 75 mass% or more of alpha-monoglucosyl hesperidin, and contains hesperidin.

As the alpha-glucosyl hesperidin, alpha-monoglucosyl hesperidin is preferable. This is because since the molecular weight of alpha-monoglucosyl hesperidin is smaller than the molecular weight of alpha-polyglucosyl hesperidin, the number of molecules of alpha-monoglucosyl hesperidin per unit mass is larger, which is considered advantageous in terms of action effect.

Alpha-Monoglucosyl hesperidin can be produced by allowing a sugar hydrolase to act on alpha-polyglucosyl hesperidin and cleave it, leaving only one glucose bonded to the hesperidin (second enzyme-treated hesperidin). Examples of the sugar hydrolase include an enzyme having a glucoamylase activity of cleaving an alpha-1,4-glucoside bond into glucose units, for example, glucoamylase (EC 3.2.1.3). Note that the percentage of alpha-monoglucosyl hesperidin in alpha-glucosyl hesperidin can be adjusted by the temperature and time conditions of enzyme treatment with glucoamylase. In addition, methods for purifying and fractionating alpha-monoglucosyl hesperidin from a mixture of enzyme-treated hesperidin is known.

The presence of various alpha-glucosyl hesperidin, hesperidin, and other components contained in the enzyme-treated hesperidin can be confirmed by a chromatogram of HPLC, and the content of each component or the purity of a desired specific component can be calculated from the peak area of the chromatogram.

The method for producing alpha-glucosyl hesperidin is not particularly limited, and a known method can be used. From the viewpoint of good yield and easy production, the enzyme treatment of hesperidin described above is preferable for the production. The method for obtaining and preparing hesperidin is not particularly limited, and a compound generally produced and sold as a reagent or a purified product may be used, or a compound prepared by extraction from raw materials such as citrus (for example, mandarin or orange) peel may be used.

<Alpha-Glucosyl rutin>

Alpha-Glucosyl rutin (also referred to as alpha glucosyl rutin) is a generic term for compounds in which one or more molecules of glucose are added to the glucose residue in the rutinose residue of rutin by an alpha1 $\rightarrow$ 4 linkage. The alpha-glucosyl rutin in the present invention may be composed of only one type of the compounds having such a structure, or may be a mixture of two or more types thereof.

Alpha-Glucosyl rutin can be represented by the following formula (2). In the formula (2), n is 0 or an integer of 1 or more, for example, an integer of 1 to 19.

[Chemical formula 2]

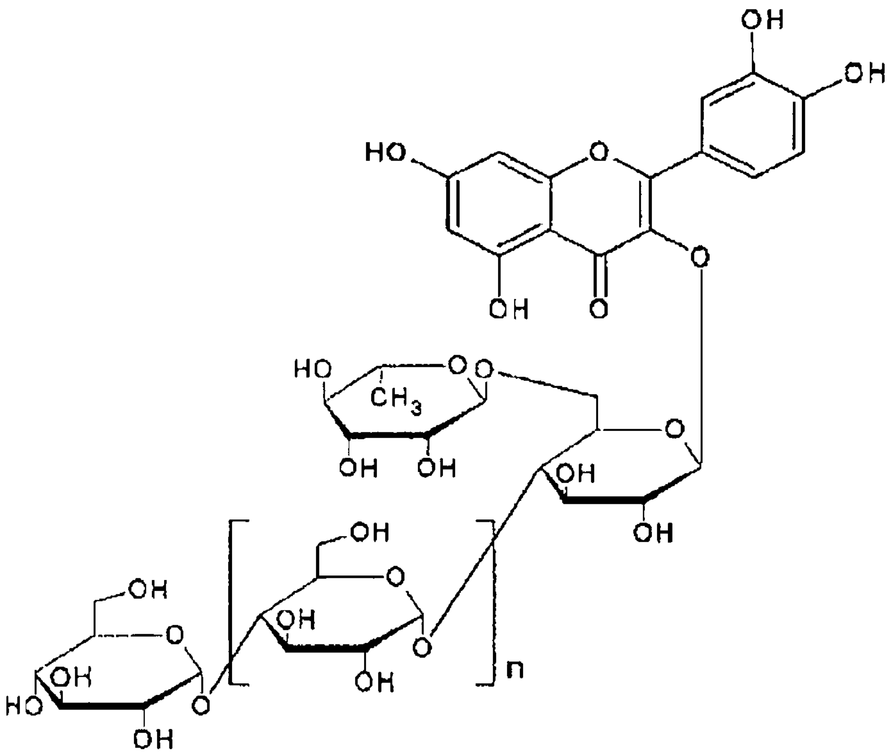

・・・(2)

Alpha-Glucosyl rutin is a compound contained as a main component in a product known as "enzyme-treated

rutin" (sometimes also referred to as "transglycosylated rutin"). Among alpha-glucosyl rutin, one to which only one glucose molecule is bonded is referred to as "alpha-monoglucosyl rutin", and one to which two or more glucose molecules are bonded is referred to as "alpha-polyglucosyl rutin". That is, in the formula (2), alpha-monoglucosyl rutin is a compound in which n is 0, and alpha-polyglucosyl rutin is a compound in which n is generally 1 to 19.

Enzyme-treated rutin is an aggregate of compounds produced by enzyme treatment for the sugar of rutin, and usually contains a mixture of compounds having different numbers of molecules of glucose bonded to rutin, for example, a mixture composed of alpha-monoglucosyl rutin and alpha-polyglucosyl rutin. In addition, since enzyme-treated rutin is generally produced by enzyme treatment, it may contain unreacted rutin and other derivatives such as isoquercitrin. Note that isoquercitrin is a compound in which beta-D-glucose is bonded to the hydroxyl group at the 3 position of the quercetin skeleton, in other words, a compound in which the rhamnose residue in the rutinose residue of rutin is cleaved.

The enzyme-treated rutin is, for example, a product obtained by allowing a glycosyltransferase (an enzyme having the function of adding glucose to rutin, such as cyclodextrin glucanotransferase (CGTase, EC 2.4.1.19)) to act on rutin in the coexistence of an alpha-glucosyl sugar compound (for example, cyclodextrin or partially decomposed starch) as a sugar donor (herein, the product is referred to as "first enzyme-treated rutin").

The first enzyme-treated rutin is a composition containing an aggregate composed of various alpha-glucosyl rutin having different numbers of molecules of bonded glucose, that is, alpha-monoglucosyl rutin and alpha-polyglucosyl rutin, and rutin that is an unreacted product. If necessary, the first enzyme-treated rutin having an increased purity of alpha-glucosyl rutin (alpha-glucosyl rutin purified product) is obtained by purifying the first enzyme-treated rutin using, for example, a porous synthetic adsorbent and an appropriate eluate to remove the sugar donor and other impurities, and further reducing the content of rutin.

In addition, the first enzyme-treated rutin is treated with an enzyme having a glucoamylase activity of cleaving alpha-1,4-glucoside bonds into glucose units, for example, glucoamylase (EC 3.2.1.3), and in alpha-glucosyl rutin to which a plurality of glucose molecules have been added, only one glucose residue directly added to the glucose residue (in the rutinose residue) of rutin itself is left and the other glucose residues are cleaved, whereby enzyme-treated rutin containing a large amount of alpha-monoglucosyl rutin (herein, referred to as "second enzyme-treated rutin") can be obtained. The glucose residue in the rutinose residue directly bonded to the quercetin skeleton is not cleaved from the quercetin skeleton by the enzyme treatment.

In the vascular endothelial function improving agent according to an embodiment of the present invention, in consideration of, for example, the effect of the present invention, enzyme-treated rutin that is a composition containing alpha-glucosyl rutin is preferably used, or a composition containing alpha-glucosyl rutin of either the first enzyme-treated rutin or the second enzyme-treated rutin may be used.

In consideration of, for example, the effect of the present invention, the enzyme-treated rutin is preferably a mixture containing at least alpha-glucosyl rutin and further containing isoquercitrin. Such a mixture can be produced by the following procedure: (i) the first enzyme-treated rutin described above is prepared, (ii) the first enzyme-treated rutin is treated with an enzyme having a glucoamylase activity to convert almost all of alpha-glucosyl rutin into alpha-monoglucosyl rutin, and (iii) the first enzyme-treated rutin is simultaneously treated with an enzyme having a rhamnosidase activity to convert almost all of unreacted rutin into isoquercitrin.

Examples of commercially available products of the enzyme-treated rutin include "alphaG rutin PS", "alphaG rutin P", and "alphaG rutin H" that are products of Toyo Sugar Refining Co., Ltd. "alphaG rutin PS" is a composition containing 65 mass% of alpha-monoglucosyl rutin and 15 mass% of isoquercitrin. "alphaG rutin P" is a composition containing 60 mass% of alpha-glucosyl rutin, 10 mass% of rutin, and 1 mass% of isoquercitrin.

As the alpha-glucosyl rutin, alpha-monoglucosyl rutin is preferable. This is because since the molecular weight of alpha-monoglucosyl rutin is smaller than the molecular weight of alpha-polyglucosyl rutin, the number of molecules of alpha-monoglucosyl rutin per unit mass is larger, which is considered advantageous in terms of action effect.

The presence of various alpha-glucosyl rutin and other components contained in the enzyme-treated rutin can be confirmed by a chromatogram of HPLC, and the content of each component or the purity of a desired specific component can be calculated from the peak area of the chromatogram.

The method for producing alpha-glucosyl rutin is not particularly limited, and a known method can be used. From the viewpoint of good yield and easy production, the enzyme treatment of rutin as described above is preferable for production. The method for obtaining and preparing rutin is not particularly limited, and a compound generally produced and sold as a reagent or a purified product may be used, or a compound prepared by extraction from raw materials such as citrus (for example, mandarin or orange) peel or buckwheat seed may be used.

<Vascular endothelial function improving agent>

A vascular endothelial function improving agent according to an embodiment of the present invention contains alpha-glucosyl hesperidin and alpha-glucosyl rutin, and preferably contains alpha-monoglucosyl hesperidin and alpha-monoglucosyl rutin from the viewpoint of a more excellent effect of improving the vascular endothelial function by a

synergistic effect of alpha-glucosyl hesperidin and alpha-glucosyl rutin. In addition to these components, as long as the effect of improving the vascular endothelial function is not impaired, the vascular endothelial function improving agent may further contain known optional components such as an excipient, a lubricant, a stabilizer, a binder, a sweetener, a disintegrant, a wetting agent, a coloring agent, a coating agent, and an emulsifier.

From the viewpoint of a more excellent effect of improving the vascular endothelial function by the synergistic effect of alpha-glucosyl hesperidin and alpha-glucosyl rutin, as for alpha-glucosyl hesperidin and alpha-glucosyl rutin in the vascular endothelial function improving agent, the content of alpha-monoglucosyl rutin is preferably 20 to 500 parts by mass relative to 100 parts by mass of alpha-monoglucosyl hesperidin. The lower limit of the content of alpha-monoglucosyl rutin relative to 100 parts by mass of alpha-monoglucosyl hesperidin is more preferably 20 parts by mass, still more preferably 65 parts by mass, and particularly preferably 120 parts by mass. The upper limit of the content of alpha-monoglucosyl rutin relative to 100 parts by mass of alpha-monoglucosyl hesperidin is more preferably 500 parts by mass, still more preferably 350 parts by mass, and particularly preferably 200 parts by mass. Within the above ranges of the content, a range having an arbitrarily combination of the lower limit and the upper limit can be arbitrarily set.

The contents of alpha-glucosyl hesperidin and alpha-glucosyl rutin in the vascular endothelial function improving agent are not particularly limited. For example, the lower limit of the content of alpha-glucosyl hesperidin or alpha-glucosyl rutin in the vascular endothelial function improving agent is, for example, 1 mass%, 2 mass%, 5 mass%, 10 mass%, or 15 mass%. In addition, the upper limit of the content of alpha-glucosyl hesperidin or alpha-glucosyl rutin in the vascular endothelial function improving agent is, for example, 99 mass%, 98 mass%, 95 mass%, 90 mass%, or 85 mass%. As for the range of the content of alpha-glucosyl hesperidin or alpha-glucosyl rutin in the vascular endothelial function improving agent, a range having an arbitrarily combination of the lower limit and the upper limit can be arbitrarily determined, and the range can be determined, for example, as 1 to 99 mass%, 2 to 98 mass%, or 5 to 95 mass%. However, the total content of alpha-glucosyl hesperidin and alpha-glucosyl rutin shall not exceed 100 mass%. Within the above range, the content of alpha-monoglucosyl rutin is more preferably 20 to 500 parts by mass relative to 100 parts by mass of alpha-monoglucosyl hesperidin.

The vascular endothelial function improving agent has an effect of improving the vascular endothelial function, which is a function of vascular endothelial cells, and thus is useful for maintaining flexibility of blood vessels that decreases with aging.

The effect of improving the vascular endothelial function can be evaluated, for example, by an FMD test as described later in the section of Examples. The effect of improving the vascular endothelial function varies depending on the amount and period of ingestion of alpha-glucosyl hesperidin and alpha-glucosyl rutin, and can be evaluated by the increase rate (%) of the FMD value (%) after ingestion of the vascular endothelial function improving agent from the FMD value (%) at the start of ingestion thereof. As for the effect of improving the vascular endothelial function, for example, the increase rate is preferably more than 100%, and more preferably 140% or more.

In addition, the effect of improving vascular endothelial function can also be evaluated from the change in FMD value (%) obtained by subtracting the FMD value (%) at the start of intake from the FMD value (%) after intake. For example, when alpha-glucosyl hesperidin and alpha-glucosyl rutin are used in combination, if there is a difference in the change compared to placebo, the use of alpha-glucosyl hesperidin alone, or the use of alpha-glucosyl rutin alone, this indicates an effect of improving vascular function, and if there is a particularly significant difference, this indicates an excellent effect of improving vascular endothelial function.

The amount of ingestion of the vascular endothelial function improving agent can be appropriately selected according to, for example, the age, gender, and race of the person who ingests the agent, and for example, the amount is preferably 17 to 1000 mg, and more preferably 70 to 500 mg per day in terms of alpha-monoglucosyl hesperidin. In addition, the amount is preferably 20 to 500 mg, and more preferably 65 to 350 mg per day in terms of alpha-monoglucosyl rutin. Regarding the number of times of ingestion of the vascular endothelial function improving agent, the above daily dose can be divided into, for example, one to three times, or two or three times. The administration period of the vascular endothelial function improving agent may be appropriately selected according to, for example, the age, gender, and race of the person who ingests the agent, and is preferably 14 to 84 days, and more preferably 28 to 56 days.

The vascular endothelial function improving agent may be ingested as it is, or may be ingested, as described later, as a food product containing the vascular endothelial function improving agent.

<Food product containing vascular endothelial function improving agent>

An embodiment of the present invention is a food product containing the vascular endothelial function improving agent, and the food product includes a beverage. Examples of the food product include beverages such as fruit beverages, oolong tea, green tea, black tea, cocoa, vegetable juice, green juice, soy milk, milk beverages, lactic acid beverages, soft drink with minute amounts of flavoring, sports drinks, and nutritional drinks; western confectionery and Japanese confectionery such as jellies, puddings, candies, gummies, gums, tablet confectionery (Ramune candies), and yogurts; seasonings; fish meat processed products; livestock processed products; foods with health claims such as foods for

specified health uses, foods with nutrient function claims, and foods with function claims; and other so-called health foods, supplements, feeds, and pet foods.

A food product containing a vascular endothelial function improving agent can be produced by adding a vascular endothelial function improving agent according to a commonly used method. The vascular endothelial function improving agent may be added at the initial stage of the production process of the food product, or may be added at the middle or final stage of the production process, and the method of addition may be appropriately selected from methods such as mixing, kneading, dissolution, immersion, dispersion, spraying, and application depending on the aspect of the food product.

The amount of the vascular endothelial function improving agent to be added to a food product is not particularly limited, but from the viewpoint of ease of ingestion and stability in the food product, the amount is 0.014 to 25 mass%, and preferably 0.14 to 12.5 mass% in terms of alpha-glucosyl hesperidin, and is 0.024 to 25 mass%, and preferably 0.24 to 21.6 mass% in terms of alpha-glucosyl rutin.

Examples

Hereinafter, a more specific description is made of the present invention with reference to examples, but the present invention is not limited to the examples, and can be appropriately modified and implemented without changing the gist thereof.

For the measurement of the FMD value (%) in the examples and comparative examples, an FMD test device (product name: UNEXEF-18VG) manufactured by UNEX Corporation was used. The measurement was conducted in accordance with a method of international guidelines for the measurement of FMD value (Corretti MC, Anderson TJ, Benjamin EJ, Celermajer D, Charbonneau F, Creager MA, et al., Guidelines for the ultrasound evaluation of endothelial-dependent flow-mediated vasodilation of the brachial artery: a report of the International Brachial Artery Reactivity Task Force., J Am Coll Cardiol 2002; 39(2): 257-65). Specifically, after measurement of the diameter of the brachial artery at rest, the cuff was inflated to 200 mmHg and maintained for 5 minutes. The cuff was then rapidly deflated, and the diameter of the artery was continuously monitored. The FMD value (%) was calculated according to the following formula.

FMD value (%) = (maximum diameter - resting diameter) $\times$ 100/resting diameter

[Example 1]

In order to evaluate the synergistic effect of combinational use of alpha-glucosyl hesperidin (alphaG hesperidin) and alpha-glucosyl rutin (alphaG rutin), an open-label, before-and-after trial was conducted as shown below.

A group of 9 healthy adult male and female subjects (8 males and 1 female) shown in the following Table 1 ingested 100 mg of alphaG hesperidin PA-T (containing 75 mass% of alpha-glucosyl hesperidin per dried product of alphaG hesperidin PA-T, manufactured by Toyo Sugar Refining Co., Ltd.) and 200 mg of alphaG rutin PS (containing 65 mass% of alpha-glucosyl rutin per dried product of alphaG rutin PS, manufactured by Toyo Sugar Refining Co., Ltd.) once a day with water or warm water on an empty stomach. The ingestion was conducted for 4 weeks, and the FMD value (%) was measured immediately after the first ingestion and immediately after the 4-week ingestion. The obtained FMD value (%) immediately after the first ingestion was defined as "initial FMD value (%)", the FMD value (%) immediately after the 4-week ingestion was defined as "FMD value (%) after 4 weeks", and the amount of change in FMD value (%) after the 4-week ingestion (that is, FMD value (%) after 4 weeks - initial FMD value (%)) was calculated. The results are shown in Fig. 1(A). The p value was calculated by t-test using 100 mg of alphaG hesperidin PA-T of Comparative example 1 described later as a control, and $p < 0.05$ was defined as having a significant difference.

The increase/decrease rate (%) of FMD value (%) after 4 weeks relative to the initial FMD value (%) taken as 100 was calculated. The results are shown in Fig. 1(B).

[Comparative example 1]

The amount of change in FMD value (%) and the increase/decrease rate (%) were calculated in the same manner as in Example 1 except that a group of 8 healthy adult male and female subjects (7 males and 1 female) shown in the following Table 1 ingested 100 mg of alphaG hesperidin PA-T (containing 75 mass% per dried product of alpha-glucosyl hesperidin, manufactured by Toyo Sugar Refining Co., Ltd.) instead of ingesting 100 mg of alphaG hesperidin PA-T and 200 mg of alphaG rutin PS. The results are shown in Fig. 1(A) and Fig. 1(B).

[Comparative example 2]

The amount of change in FMD value (%) and the increase/decrease rate (%) were calculated in the same manner as in Example 1 except that a group of 7 healthy adult male and female subjects (5 males and 2 females) shown in the

following Table 1 ingested 200 mg of alphaG rutin PS instead of ingesting 100 mg of alphaG hesperidin PA-T and 200 mg of alphaG rutin PS. The results are shown in Fig. 1(A) and Fig. 1(B).

[Comparative example 3]

The amount of change in FMD value (%) and the increase/decrease rate (%) were calculated in the same manner as in Example 1 except that a group of 8 healthy adult male and female subjects (7 males and 1 female) shown in the following Table 1 ingested 300 mg of alphaG rutin PS instead of ingesting 100 mg of alphaG hesperidin PA-T and 200 mg of alphaG rutin PS. The results are shown in Fig. 1(A) and Fig. 1(B).

[Comparative example 4]

The amount of change in FMD value (%) and the increase/decrease rate (%) were calculated in the same manner as in Example 1 except that a group of 4 healthy adult male and female subjects (4 males and 0 females) shown in the following Table 1 ingested 300 mg of alphaG hesperidin PA-T instead of ingesting 100 mg of alphaG hesperidin PA-T and 200 mg of alphaG rutin PS. The results are shown in Fig. 1(A) and Fig. 1(B).

[Table 1]

| | Amount of ingestion | | Group of subjects | | |
|---|---|---|---|---|---|
| | alphaG hesperidin PA-T | alphaG rutin PS | Age | BMI | Initial FMD value (%) |
| Example 1 | 100 mg | 200 mg | 42.9 ± 3.0 | 21.8 ± 0.9 | 4.56 ± 0.67 |
| Comparative example 1 | 100 mg | | 43.4 ± 3.2 | 22.5 ± 0.8 | 4.93 ± 0.83 |
| Comparative example 2 | | 200 mg | 42.7 ± 2.9 | 21.9 ± 0.4 | 4.94 ± 0.57 |
| Comparative example 3 | | 300 mg | 41.6 ± 2.7 | 21.3 ± 1.0 | 5.39 ± 0.77 |
| Comparative example 4 | 300 mg | | 42.5 ± 7.9 | 22.5 ± 2.1 | 4.63 ± 1.60 |
| Mean value ± standard deviation | | | | | |

[Example 2]

Next, in order to evaluate the synergistic effect of combinational use of alphaG hesperidin and alphaG rutin, a double-blind, parallel group trial was conducted as described below.

A group of 12 healthy adult male and female subjects (3 males and 9 females) shown in the following Table 2 ingested 100 mg of alphaG hesperidin PA-T and 200 mg of alphaG rutin PS once a day with water or warm water on an empty stomach. The ingestion was conducted for 8 weeks, and the FMD value (%) was measured immediately after the first ingestion and immediately after the 8-week ingestion. The obtained FMD value immediately after the first ingestion was defined as "initial FMD value (%)", the FMD value (%) immediately after the 8-week ingestion was defined as "FMD value (%) after 8 weeks", and the amount of change in FMD value (%) after the 8-week ingestion (that is, FMD value (%) after 8 weeks - initial FMD value (%)) was calculated. The results are shown in Fig. 2. The p value was calculated by t-test using a placebo described later as a control, and $p < 0.05$ was defined as having a significant difference.

In addition, a group of 9 healthy adult male and female subjects (2 males and 7 females), that is, a placebo group ingested a placebo containing 200 mg of partially pregelatinized starch and 75 mg of sugar alcohol instead of alphaG hesperidin PA-T and alphaG rutin PS.

[Comparative example 5]

The amount of change in FMD value (%) was calculated in the same manner as in Example 2 except that a group of 9 healthy adult male and female subjects (3 males and 6 females) shown in the following Table 2 ingested 100 mg of alphaG hesperidin PA-T instead of ingesting 100 mg of alphaG hesperidin PA-T and 200 mg of alphaG rutin PS. The results are shown in Fig. 2.

[Table 2]

| | Amount of ingestion | | Group of subjects | | |
|---|---|---|---|---|---|
| | alphaG hesperidin PA-T | alphaG rutin PS | Age | BMI | Initial FMD value (5%) |
| Example 2 | 100 mg | 200 mg | 51.3 ± 8.8 | 23.7 ± 3.3 | 6.1 ± 2.2 |
| Comparative example 5 | 100 mg | | 56.2 ± 11.1 | 25.7 ± 1.6 | 6.0 ± 1.0 |
| Placebo group | | | 53.2 ± 9.3 | 25.4 ± 3.1 | 6.0 ± 2.5 |
| Mean value ± standard deviation | | | | | |

From the results in Fig. 1 and Fig. 2, it was found that the FMD value (%) was improved in the case where alphaG hesperidin and alphaG rutin were ingested in combination, as compared with the case where alphaG hesperidin was ingested alone and the case where alphaG rutin was ingested alone. Therefore, it was shown that the effect of improving the vascular endothelial function is obtained by the synergistic effect of alphaG hesperidin and alphaG rutin.

## Claims

**1.** A vascular endothelial function improving agent comprising:

alpha-glucosyl hesperidin; and
alpha-glucosyl rutin.

**2.** The vascular endothelial function improving agent according to claim 1, comprising:

alpha-monoglucosyl hesperidin; and
alpha-monoglucosyl rutin.

**3.** The vascular endothelial function improving agent according to claim 2, wherein a content of the alpha-monoglucosyl rutin is 20 to 500 parts by mass relative to 100 parts by mass of the alpha-monoglucosyl hesperidin.

**4.** A food product comprising the vascular endothelial function improving agent according to claim 1.

[Fig. 1]

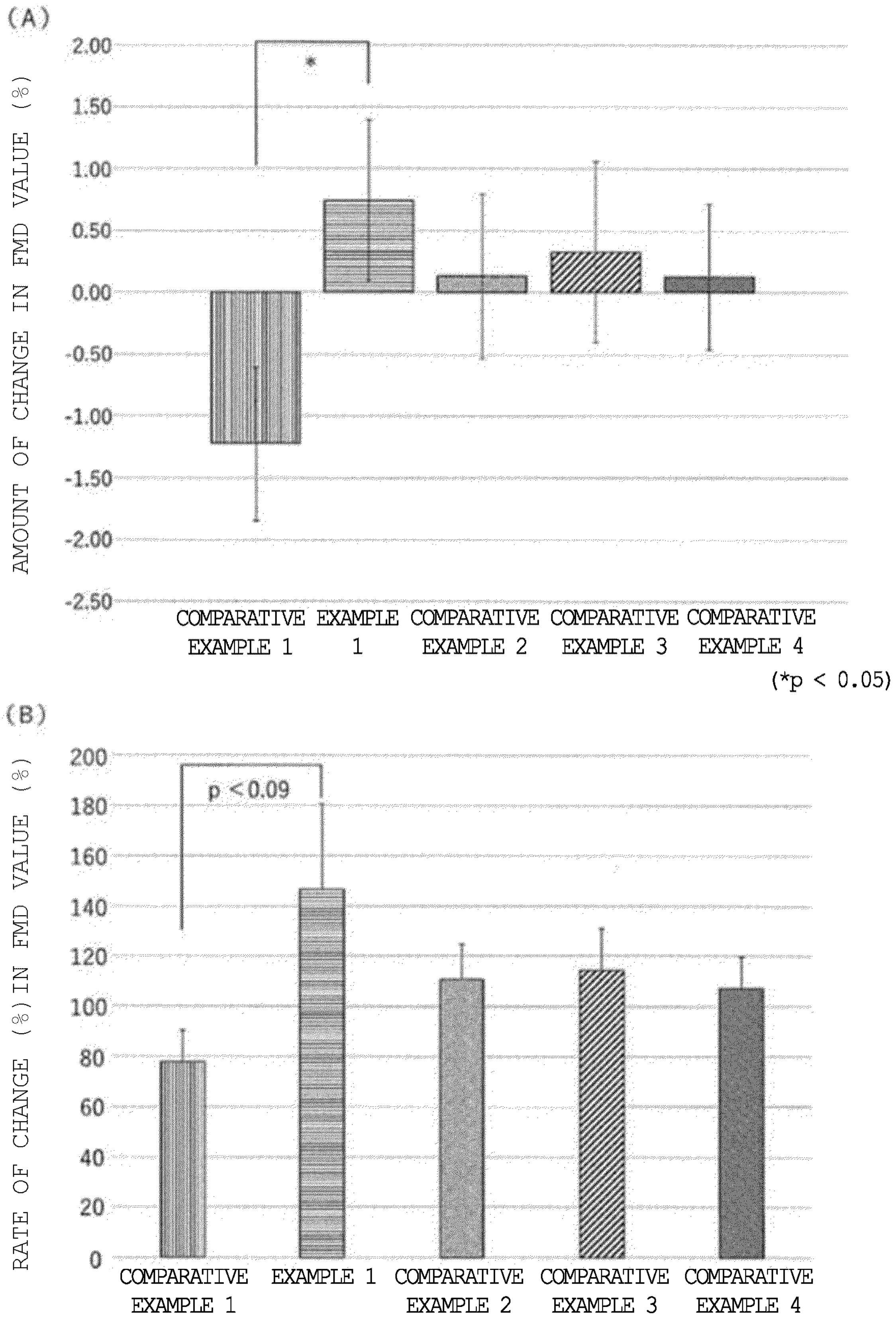
(A)
AMOUNT OF CHANGE IN FMD VALUE (%)
2.00
1.50
1.00
0.50
0.00
-0.50
-1.00
-1.50
-2.00
-2.50
*
COMPARATIVE EXAMPLE 1
EXAMPLE 1
COMPARATIVE EXAMPLE 2
COMPARATIVE EXAMPLE 3
COMPARATIVE EXAMPLE 4
(*p < 0.05)
(B)
RATE OF CHANGE (%) IN FMD VALUE (%)
200
180
160
140
120
100
80
60
40
20
0
p < 0.09
COMPARATIVE EXAMPLE 1
EXAMPLE 1
COMPARATIVE EXAMPLE 2
COMPARATIVE EXAMPLE 3
COMPARATIVE EXAMPLE 4

[Fig. 2]

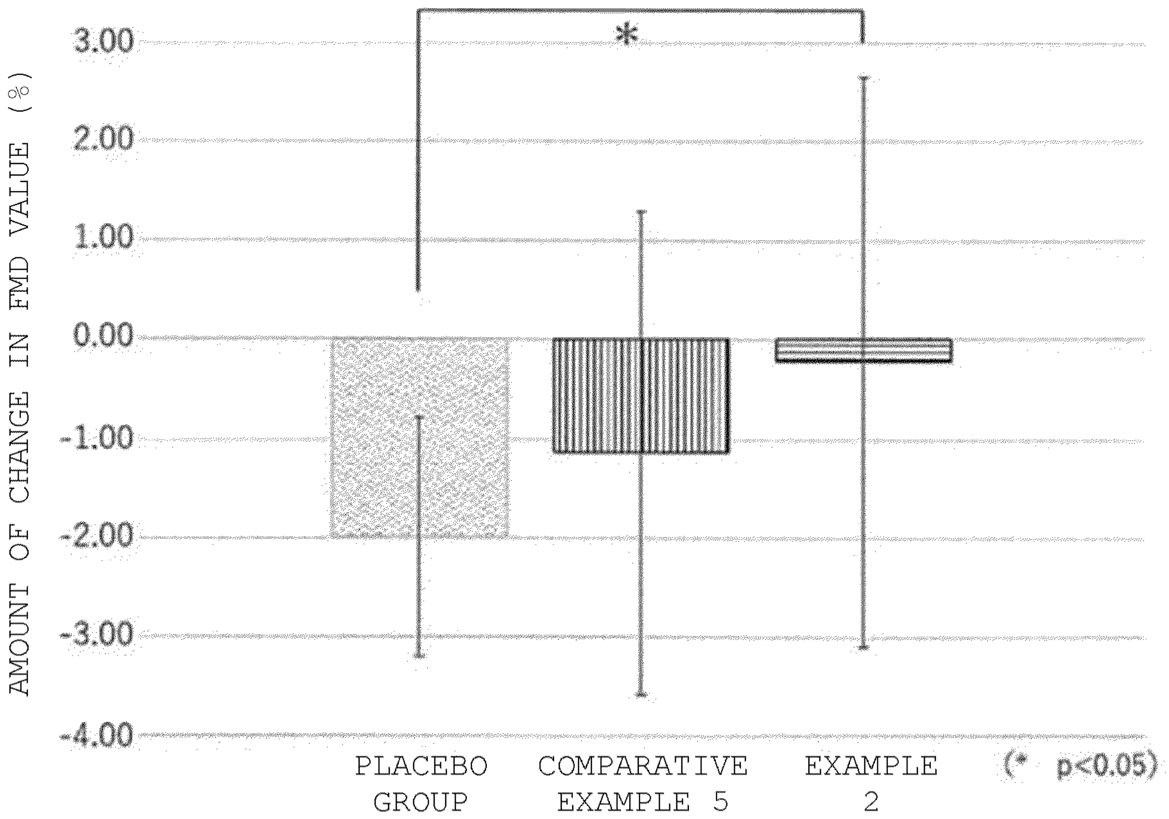
*
3.00
2.00
1.00
0.00
-1.00
-2.00
-3.00
-4.00
AMOUNT OF CHANGE IN FMD VALUE (%)
PLACEBO GROUP
COMPARATIVE EXAMPLE 5
EXAMPLE 2
(* p<0.05)

**INTERNATIONAL SEARCH REPORT**

International application No.
**PCT/JP2024/006981**

**A. CLASSIFICATION OF SUBJECT MATTER**

***A23L 33/10***(2016.01)i; ***A61K 31/7048***(2006.01)i; ***A61P 9/00***(2006.01)i
FI: A23L33/10; A61K31/7048; A61P9/00

According to International Patent Classification (IPC) or to both national classification and IPC

**B. FIELDS SEARCHED**

Minimum documentation searched (classification system followed by classification symbols)

A23L33/10; A61K31/7048; A61P9/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X<br>Y | JP 2013-018731 A (HAYASHIBARA, Masaaki) 31 January 2013 (2013-01-31)<br>test example 5, paragraph [0034] | 1-4<br>3 |
| A | 宅見央子ほか. 糖転移ヘスペリジンの血流改善作用. 応用糖質科学. 2011 (issue date), vol. 1, no. 2, pp. 186-193, (TAKUMI, Hiroko et al. Effects of α-Glucosyl Hesperidin on the Blood Circulation. Journal of Applied Glycoscience.) | 1-4 |
| A | 西田清一郎ほか. 漢方生薬含有機能性フラボノイド"ケルセチン"の血管薬理作用. 日薬理誌. vol. 146, 2015, pp. 140-143, (NISHIDA, Seiichiro et al. The vascular pharmacological effects induced by quercetin contained in Kampo herbal medicine. Folia Pharmacologica Japonica.) | 1-4 |
| A<br>Y | JP 2015-117213 A (KAO CORPORATION) 25 June 2015 (2015-06-25)<br>paragraph [0030] | 1-2, 4<br>3 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **09 May 2024** | **21 May 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**

International application No.
**PCT/JP2024/006981**

**C. DOCUMENTS CONSIDERED TO BE RELEVANT**

| Category* | Citation of document, with indication, where appropriate, of the relevant passages | Relevant to claim No. |
|---|---|---|
| X<br>Y | WO 2015/083554 A1 (KABUSHIKI KAISHA HAYASHIBARA) 11 June 2015 (2015-06-11) example 21, paragraphs [0003], [0004] | 1-2, 4<br>3 |
| X<br>Y | JP 2008-061508 A (MITSUI SUGAR CO., LTD.) 21 March 2008 (2008-03-21) example 1d, paragraph [0040] | 1-2, 4<br>3 |
| A | JP 2008-297279 A (KAO CORPORATION) 11 December 2008 (2008-12-11) | 1-4 |
| A | WO 2020/162532 A1 (SAN-EI GEN F. F. I., INC.) 13 August 2020 (2020-08-13) | 1-4 |

Form PCT/ISA/210 (second sheet) (July 2022)

**INTERNATIONAL SEARCH REPORT**
**Information on patent family members**

International application No.
**PCT/JP2024/006981**

| Patent document cited in search report | Publication date (day/month/year) | Patent family member(s) | Publication date (day/month/year) |
|---|---|---|---|
| JP 2013-018731 A | 31 January 2013 | (Family: none) | |
| JP 2015-117213 A | 25 June 2015 | (Family: none) | |
| WO 2015/083554 A1 | 11 June 2015 | (Family: none) | |
| JP 2008-061508 A | 21 March 2008 | (Family: none) | |
| JP 2008-297279 A | 11 December 2008 | (Family: none) | |
| WO 2020/162532 A1 | 13 August 2020 | US 2022/0079967 A1<br>EP 3922252 A1 | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 4295840 B
- WO 2009116450 A1


### Non-patent literature cited in the description


- **CORRETTI MC** ; **ANDERSON TJ** ; **BENJAMIN EJ** ; **CELERMAJER D** ; **CHARBONNEAU F** ; **CREAGER MA et al.** Guidelines for the ultrasound evaluation of endothelial-dependent flow-mediated vasodilation of the brachial artery: a report of the International Brachial Artery Reactivity Task Force.. *J Am Coll Cardiol*, 2002, vol. 39 (2), 257-65